# EUROPÄISCHE PATENTSCHRIFT

(11) **EP 0 238 065 B1**
(45) Veröffentlichungstag und Bekanntmachung des Hinweises auf die Patenterteilung: **14.09.1994**
(21) Anmeldenummer: 87103981.4
(22) Anmeldetag: 18.03.1987
(51) Int. Cl.: A61B 5/02

(54) **Mikroprozessorgesteuerte Einrichtung zur nichtinvasiven Feststellung peripherer Abfluss- und Durchflussstörungen**
Microprocessor-controlled device for non-invasively detecting anomalies of peripheral blood circulation
Dispositif commandé par microprocesseur et destiné à la détection non envahissante d'anomalies de la circulation sanguine périphérique

(30) Priorität: 18.03.1986 DE 3609075
(43) Veröffentlichungstag der Anmeldung: 23.09.1987
(73) Patentinhaber: A. Nattermann & Cie. GmbH, D-50829 Köln (DE)
(72) Erfinder: Schmitt, Hans J., Prof. Dr. rer. nat., D-5100 Aachen (DE); Blazek, Vladimir, Dr.-Ing., D-5100 Aachen (DE)
(74) Vertreter: Schiller, Walter, Dr.

(56) Entgegenhaltungen:
- EP-A- 0 063 649
- DE-A- 3 409 792
- GB-A- 2 071 339
- US-A- 4 166 454
- US-A- 4 367 752
- OFS THIRD INTERNATIONAL CONFERENCE ON OPTICAL FIBER SENSORS, San Diego, California, 13.-14. Februar 1985, Seiten 136,137; H.J. SCHMITT et al.: "Thee 2: fiber optical sensor for medical applications"

## Beschreibung

Die Erfindung bezieht sich auf eine Meßeinrichtung zur nichtinvasiven Feststellung peripherer Abfluß-und Durchflußstörungen in menschlichen Extremitäten gemäß dem Oberbegriff des Patentanspruchs 1.

Eine gattungsgemäße Meßeinrichtung ist aus der US-A-4 166 454 bekannt. Bei dieser Meßeinrichtung handelt es sich um einen sogenannten Cardiac-Monitor, der die Herzaktivität und insbesondere den systolischen Puls durch Messung und Auswertung der Anderung der Lichtreflexion der Arterien erfassen soll. Bei dieser Meßeinrichtung wird nach dem Einschalten der Strom des Lichtsenders so lange erhöht, bis ein nomineller Stromwert, also ein über die Signal-und die Rauschanteile summierender Stromwert des Phototransistors erreicht wird. Damit ist jedoch nicht gewährleistet, daß das Meßsignal einen bestimmten minimalen Signal/Rausch-Abstand hat.

Weitere Meßeinrichtungen zur nichtinvasiven Feststellung peripherer Abfluß-und Durchflußstörungen in menschlichen Extemitäten sind aus der DE-C-31 00 610 und der DE-C-33 18 746 bekannt. Bei der aus der DE-C-31 00 610 bekannten Meßeinrichtung wir der zeitliche Verlauf des reflektierten bzw. rückgestreuten Strahlungsanteils analog ausgewertet und mittels eines Schreibers aufgezeichnet.

Bei der aus der DE-C-33 18 746 bekannten Meßeinrichtung wird das analoge Signal mittels einer Schnittstellenschaltung in ein digitales Signal umgesetzt und an einen Rechner angelegt. Der Rechner berechnet physikalische Bewertungsparameter für die analogen Lichtreflexions-Kurven.

Diesen bekannten Meßeinrichtungen ist der Nachteil gemeinsam, daß sie kaum als handliches, tragbares Gerät vor allem deshalb ausführbar sind, weil der Stromverbrauch dieser Einrichtungen für tragbare Geräte zu groß ist.

Ein tragbares Gerät anderer Gattung, das insbesondere als Pulsmesser verwendet wird, ist aus der DE-A-3409 792 bekannt. Dieses Gerät erlaubt jedoch nicht die nichtinvasive Feststellung peripherer Abfluß-und Durchflußstörungen in menschlichen Extremitäten durch Messung der Änderung der Lichtreflexion.

Der Erfindung liegt die Aufgabe zugrunde, eine Meßeinrichtung zur nichtinvasiven Feststellung peripherer Abfluß-und Durchflußstörungen in menschlichen Extremitäten, die zur Erfassung des zeitlichen Verlaufs der Blutentleerung bzw. Auffüllung von Adern die Änderung der Lichtreflexion mißt, anzugeben, deren Energieverbrauch dadurch minimiert ist, daß der Signal/Rausch-Abstand gerade einen bestimmten, für die Messung noch ausreichenden Wert hat, so daß die Realisierung eines kompakten und leichen Gerätes möglich ist.

Eine erfindungsgemäße Lösung dieser Aufgabe ist im Patentanspruch 1 angegeben. Weiterbildungen der Erfindung sind Gegenstand der abhängigen Ansprüche.

Erfindungsgemäß liegt zur Erfassung des zeitlichen Verlaufs der Blutentleerung und -auffüllung der Venen an der Regelschleife als Führungsgröße der Signal/Rausch-Abstand des Ausgangssignals des bzw. der Lichtempfänger an. Die Pegelsteuereinheit steuert den oder die Lichtsender derart an, daß der durch den bzw. die Lichtsender fließende Strom vor Beginn der eigentlichen Messung solange erhöht wird, bis der Signal/Rausch-Abstand gerade den bestimmten, für die Messung noch, ausreichenden Wert erreicht hat. Dieser Stromwert wird dann während der Messung beibehalten.

Durch diesen erfindungsgemäßen Aufbau werden die Lichtsender immer mit einer "gerade ausreichenden" Leistung beaufschlagt, so daß unnötiger Energieverbrauch - was gerade für ein Handgerät wichtig ist - vermieden wird.

Dieser erfindungsgemäße Grundgedanke erlaubt es sogar, den Meßkopf, der auf die Haut aufgesetzt wird, mit dem eigentlichen Handgerät über eine Lichtleitfaser zu verbinden, wie dies beispielsweise in dem Konferenzbericht OFS Third Int. Conf. on Optical Fibre Sensors, San Diego, Calif.; 13-14 Feb. 1985, Seiten 136, 137; H.J. Schmitt et al.: "Thee 2: Fibre optical sensor for medical applications" beschrieben ist. Bei einer nichtaktiven Regelung ist eine derartige Verbindung über eine Lichtleitfaser aufgrund ihrer wechselnden Verluste und des damit schwankenden Ausgangssignals kritisch.

Der erfindungsgemäße Grundgedanke erlaubt damit den Aufbau eines kleinen, tragbaren - sogar in einer Hand zu haltenden - Geräts, das durch Batterien oder wiederaufladbare Akkumulatoren gespeist werden kann.

Bei einer bevorzugten Weiterbildung der Erfindung, die im Anspruch 2 gekennzeichnet ist, weist die Auswerte-und Ausgabeschaltung eine Aktivregelschleife für den durch den oder die Lichtsender fließenden Strom auf, die von einem Mikroprozessor gesteuert wird.

Die Ausbildung der erfindungsgemäßen Einrichtung gemäß den Ansprüchen 3 bzw. 7 befreit die Messung von subjektiven Meßfehlern, die durch eine unrichtige Festlegung des Meßbeginns und des Meßendes entstehen.

Die im Anspruch 4 beanspruchte vorteilhafte Weiterbildung ermöglicht - in an sich bekannter Weise - eine leichte Trennung des Meßsignals von der Umgebungsbeleuchtung, und damit eine weitere Herabsetzung des Leistungsbedarfs der erfindungsgemäßen Einrichtung.

In den Ansprüchen 5 und 6 sind - an und für sich aus der US-A-4 367 752 bekannte Möglichkeiten angegeben, die Ausgabe der erhaltenen Meßergebnisse stromsparend mit dem Handgerät durchzuführen. Die im Anspruch 5 beschriebene LCD-Anzeigeeinheit kann beispielsweise eine acht- oder mehrstellige alphanumerische LCD-Anzeige oder eine LCD-Anzeige sein, die eine punktweise Ansteuerung und damit die Darstellung von Graphiken etc. erlaubt.

Die Ausgabe der Meßergebnisse über einen elektroakustischen Wandler, beispielsweise einem Lautsprecher oder einem Kopfhörer, die im Anspruch 6 angeführt ist, ist in der am gleichen Tag eingereichten Patentanmeldung "Einrichtung zur nichtinvasiven Feststellung und akustischen Darstellung des dynamischen Verhaltens der peripheren venösen Hämodynamik" im einzelnen beschrieben. Diese Art der Ausgabe der Meßergebnisse ist nicht nur stromsparend, sondern gibt dem Artz auch einen bequemen Überblick über den Gefäßzustand.

Die im Anspruch 8 gekennzeichnete Ausbildung sorgt für eine weitere Verringerung des Stromverbrauchs.

Die Auswerte- und Ausgabeeinheit kann - wie ebenfalls erfindungsgemäß erkannt worden ist - zusätzlich verschiedene Bewegungsprogramme in einem Speicher speichern, die sie beispielsweise akustisch ausgibt (Anspruch 9).

Darüberhinaus kann die Auswerten und Ausgabeeinheit bei akustischer Ausgabe der Meßergebnisse auch eine Tonfolge speichern, die der Untersuchungsperson das "Lernen" der Interpretation der akustischen Ausgabe erlaubt.

Die Erfindung wird nachstehend anhand eines Ausführungsbeispiels unter Bezugnahme auf die Zeichnung näher beschrieben,
in der zeigen:
- Fig. 1: ein Blockschaltbild der erfindungsgemäßen Auswerte und Ausgabeeinheit,
- Fig. 2: ein Blockschaltbild eines Ausführungsbeispiels des Mikoprozessor-Systems, und
- Fig. 3: typische Meßergebnisse.

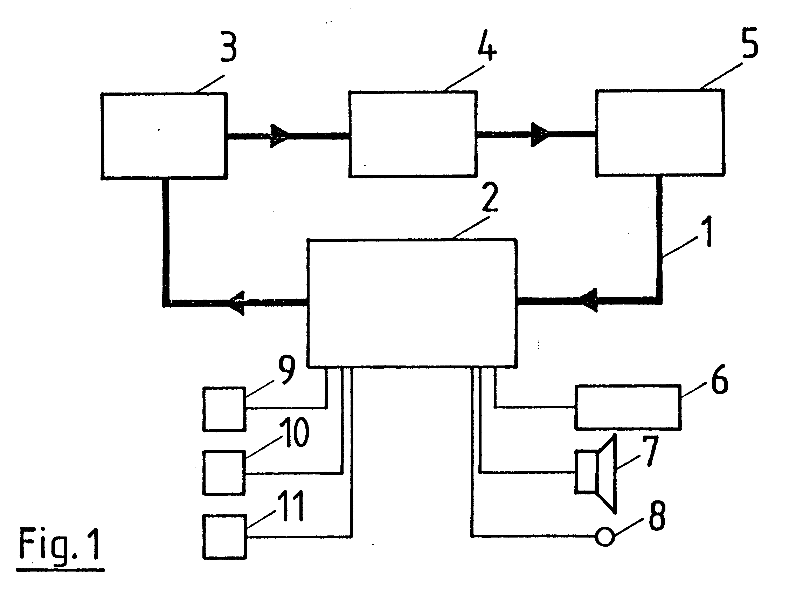

Fig. 1 zeigt ein Blockschaltbild eines Ausführungsbeispiels der erfindungsgemäßen Auswerte- und Ausgabeeinheit. Eine aktive Regelschleife (1) für den durch die Lichtsender fließenden Strom wird durch ein Mikoprozessor-System (2), eine Treiberstufe und Pegelsteuereinheit (3) für den oder die Lichtsender, den optischen Meßkopf (4) mit einem oder mehreren Lichtsendern und einem oder mehreren Lichtempfängern, und einer Empfangs- und Demodulatoreinheit (5) für die Signale des oder der Lichtempfänger gebildet.

Die Treiberstufe (3) versorgt in an sich bekannter Weise den oder die Lichtsender mit einem gepulsten Strom, dessen "Mittelwert" durch die aktive Regelschleife (1) derart gesteuert wird, daß der vom Mikroprozessorsystem kontrollierte Signal/Rauschabstand am Ausgangsanschluß der Einheit (5) "gerade" einen bestimmten Wert erreicht.

Ferner sind eine LCD-Anzeigeeinheit (6), ein elektroakustischer Wandler (7), beispielsweise ein Lautsprecher, ein Datensignal-Ausgangsanschluß (8), der beispielsweise eine standardisierte Schnittstelle zu einem Drucker und/oder einem Rechner sein kann, ein Ein/Aus-Schalter (9), eine Taste (10) mit der verschiedene Funktionen einschaltbar sind, sowie eine Taste (11) vorgesehen, die die Bedienung der Anzeigeeinheit (6) erlaubt.

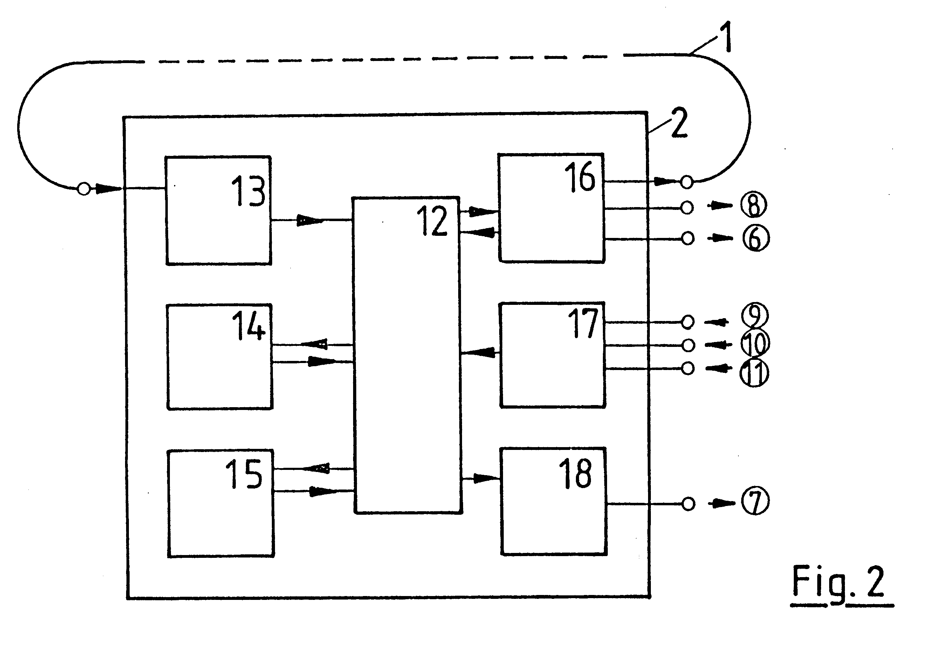

Fig. 2 zeigt im einzelnen den Aufbau eines Ausführungsbeispiels eines erfindungsgemäßen Mikroprozessor-Systems (2). Das Mikroprozessor-System (2) weist einen eigentlichen Mikroprozessor (12), einen Analog/Digital-Wandler (13), der die Ausgangssignale der Empfangs- und Demodulatoreinheit (5) wandelt, einen Programmspeicher (14) für den Mikroprozessor (12), einen Arbeits- und Datenspeicher (15), eine digitale Ausgabeeinheit (16), eine digitale Eingabeeinheit (17) sowie eine analoge Ausgabeeinheit (18) auf. Die Verbindungen der Elemente (16, 17 und 18) zu den in Fig. 1 dargestellten Elementen sind in Fig. 2 eingetragen.

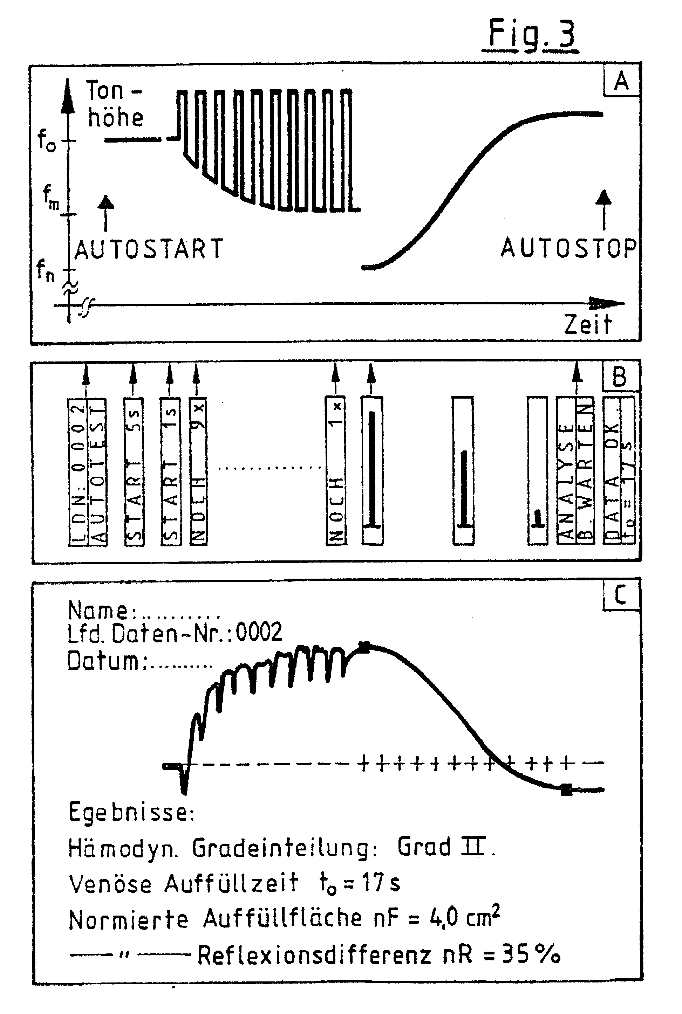

Die Funktionsweise der in den Figuren 1 und 2 schematisch dargestellten Auswerte- und Ausgabeeinheit wird im folgenden in Verbindung mit Fig. 3 näher erläutert:
Wie in der am gleichen Tag eingereichten parallelen Patentanmeldung "Einrichtung ... zur akustischen Darstellung..." näher erläutert ist, wird das gemessene Reflexionssignal mittels des Mikroprozessors (12), der analogen Ausgabeeinheit (18), die beispielsweise ein Signalverstärker sein kann, und dem Lautsprecher (7) in eine Tonfolge umgesetzt, deren Frequenz der Lichtreflexion und damit dem in den Gefäßen herrschenden Druck entspricht. Die Blutentleerung bzw. -auffüllung, für die der Druck bzw. die Lichtreflexion ein Maß ist, und die sich damit ergebende Tonhöhe ist schematisch in Fig. 3 A dargestellt. Die Tonhöhe stellt damit ein Maß für die Intensiät des Meßsignals und nicht etwa für die Frequenzverschiebung des Meßsignals dar.

Fig. 3 B zeigt die auf der LCD-Anzeigeeinheit (6) zu den jeweiligen Zeitpunkten erscheinende Anzeige. Die Anzeigeeinheit (6) enthält zu Beginn der Messung eine Information darüber, daß zur Zeit ein Selbsttest durchgeführt wird, bzw. wieviel Zeit bis zum Beginn der Messung noch vergeht. Anschließend zeigt sie die Zahl der noch während des Bewegungsprogramms zu hörenden Töne und damit die Restdauer des Bewegungsprogramms an. Während der Auffüllphase kann sie beispielsweise durch Darstellung eines analogen Balkens die momentane Hautreflexion analog anzeigen. Nach Beendigung der Auffüllphase und einer kurzen Analysephase zeigt sie z.B. die Zeitdauer tₒ an, die zur Auffüllung der Gefäße bis zu einem hämodynamischen Ruhezustand benötigt worden ist.

Fig. 3 C zeigt ein Beispiel für eine Ausgabe, die man auf einen externen Drucker erhält, der mit der erfindungsgemäßen Meßeinrichtung über den Ausgangsanschluß (8) verbunden ist. Ausgegeben werden nicht nur die Meßwerte, sondern auch die vom Mikroprozessor berechneten Parameter.

Vorstehend ist die Erfindung anhand eines Ausführungsbeispiels ohne Beschränkung der Allgemeinheit beschrieben worden. Innerhalb des allgemeinen Erfindungsgedankens sind selbstverständlich die verschiedensten Modifikationen möglich:
Beispielsweise kann die vor Beginn der Meßung durchgeführte Selbsteichung nicht nur die Einstellung eines bestimmten Signal-Rauschabstandes sowie bei akustischer Ausgabe die Einstellung einer bestimmten Tonhöhe umfassen, sondern auch einen "Null-Abgleich" der gesamten analogen Elektronik, der vom digitalen Teil durchgeführt wird.

Darüberhinaus können die im Programmspeicher des Mikroprozessor-Systems (2) niedergelegten Funktionsabläufe (Programmpakete) nicht nur den Meßmodus und einen Ausgabemodus umfassen, sondern auch ein "Lernprogramm", das die Bedienungsperson bei der akustischen Wahrnehmung bestimmter akustisch umgesetzter hämodynamischer Vorgänge (beliebig oft) schult. Die Auswahl der einzelnen Programmepakete kann dabei über die Taste (10) getroffen werden.

Ferner kann u.a. auch die Anzeige und/oder die akustische Darstellung mittels der Taste (11) abschaltbar sein, so daß gegebenenfalls eine weitere Stromersparnis erreicht wird.

In jedem Falle ermöglicht jedoch das erfindungsgemäße Prinzip den Aufbau eines kompakten und leicht zu handhabenden Geräts, das jederzeit mit einer Hand gehalten und an wechselnden Einsatzorten eingesetzt werden kann.

## Patentansprüche

1. Meßeinrichtung zur nichtinvasiven Feststellung peripherer Abfluß- und Durchflußstörungen in menschlichen Extremitäten, die zur Erfassung des zeitlichen Verlaufs der Blutentleerung bzw. -auffüllung von Adern durch Messung der Änderung der Lichtreflexion
- mindestens einen Lichtsender und mindestens einen Lichtempfänger,
- eine Regelschleife (1) zur Steuerung einer Pegelsteuereinheit für den durch den bzw. die Lichtsender fließenden Strom
- sowie eine Auswerte- und Ausgabeschaltung aufweist,
dadurch **gekennzeichnet**, daß zur Erfassung des zeitlichen Verlaufs der Blutentleerung und -auffüllung der Venen an der Regelschleife als Führungsgröße der Signal/Rauschabstand des Ausgangssignals des bzw. der Lichtempfänger anliegt, und
daß die Pegelsteuereinheit (3) den oder die Lichtsender derart ansteuert, daß der durch den bzw. die Lichtsender fließende Strom vor Beginn der eigentlichen Messung so lange erhöht wird, bis der Signal/Rausch-Abstand gerade einen bestimmten, für die Messung noch ausreichenden Wert erreicht hat, und daß dieser Stromwert während der Messung beibehalten wird.

2. Einrichtung nach Anspruch 1,
dadurch **gekennzeichnet**, daß die Auswerte- und Ausgabeschaltung eine von einem Mikroprozessor gesteuerte aktive Regelschleife für den durch den oder die Lichtsender fließenden Strom aufweist.

3. Einrichtung nach Anspruch 1 oder 2,
dadurch **gekennzeichnet**, daß die Auswerte- und Ausgabeschaltung erst dann den Start einer Messung ermöglicht, wenn ein Ruhezustand der Hautdurchblutung erfaßt wird.

4. Einrichtung nach einem der Ansprüche 1 bis 3,
dadurch **gekennzeichnet**, daß der durch den oder die Lichtsender fließende Strom mit einer bestimmten Frequenz moduliert ist, und die Auswerte- und Ausgabeschaltung das Ausgangssignal des oder, der Lichtempfänger mit der gleichen Frequenz demoduliert.

5. Einrichtung nach einem der Ansprüche 1 bis 4,
dadurch **gekennzeichnet**, daß die Auswerte- und Ausgabeschaltung eine LCD-Anzeigeeinheit aufweist.

6. Einrichtung nach einem der Ansprüche 1 bis 5,
dadurch **gekennzeichnet**, daß die Anzeige- und Auswerteschaltung zur Ausgabe der Meßergebnisse einen elektroakustischen Wandler aufweist.

7. Einrichtung nach einem der Ansprüche 1 bis 6,
dadurch **gekennzeichnet**, daß die Anzeige- und Auswerteschaltung die Messung beendet, wenn wieder ein Ruhezustand der Hautdurchblutung erfaßt wird.

8. Einrichtung nach einem der Ansprüche 1 bis 7,
dadurch **gekennzeichnet**, daß die Auswerte- und Ausgabeschaltung den Strom durch die Lichtsender nach der eigentlichen Meßphase und nach dem selbsttätigen Abschalten der Messung ganz oder teilweise abschaltet.

9. Einrichtung nach einem der Anspürche 1 bis 8,
dadurch **gekennzeichnet**, daß die Auswerte- und Ausgabeeinheit mehrere Bewegungsprogramme speichert, die sie akustisch ausgibt.

10. Einrichtung nach einem der Ansprüche 1 bis 9,
dadurch **gekennzeichnet**, daß die Auswerte- und Ausgabeeinheit eine "Lern-Tonfolge" speichert.

11. Einrichtung nach einem der Ansprüche 1 bis 10,
dadurch **gekennzeichnet**, daß der optische Meßkopf als faseroptischer Sensor ausgebildet ist.

12. Einrichtung nach einem der Ansprüche 1 bis 11,
dadurch **gekennzeichnet**, daß die Auswerte- und Ausgabeschaltung jeder Messung selbsttätig eine fortlaufende und nicht rücksetzbare Nummer zuteilt.

## Claims

1. Device for the non-invasive detection of peripheral disorders in the blood discharge and circulation in human extremities, which comprises
- at least one light emitter and at least one light receiver,
- a control loop (1) for controlling a level controller for adjusting the current flowing through said light emitter(s),
- as well as an evaluation and output circuit,
for detecting the variation, as a function of time, of blood discharge from and blood supply to blood vessels by measuring the variation of light reflection,
**characterized in** that the signal-to-noise ratio of the output signal from said light emitter(s) is applied to said control loop as a reference variable for detecting the variation versus time of the blood discharge from and blood supply to the blood vessels, and
in that said level controller (3) controls said light emitter(s) in such a way that prior to the beginning of the actual measuring cycle the current flowing through said light emitter(s) is increased until the signal-to-noise ratio has just reached a certain value still sufficient for measuring, and that this current value is maintained throughout the measurement.

2. Device according to Claim 1,
**characterized** in that said evaluation and output circuit comprises an active control loop under microprocessor control, for controlling the current flowing through said light emitter(s).

3. Device according to Claim 1 or 2,
**characterized** in that said evaluation and output circuit enables the start of a measuring cycle only if a rest status in the blood flow in the skin is detected.

4. Device according to any of Claims 1 to 2,
**characterized** in that the current flowing through said light emitter(s) is modulated with a certain frequency, and that said evaluation and output circuit demodulates the output signal from said light emitter(s) with the same frequency.

5. Device according to any of Claims 1 to 4,
**characterized** in that said evaluation and output circuit comprises an LCD display unit.

6. Device according to any of Claims 1 to 5,
**characterized** in that said display and evaluation circuit comprises an electro-acoustic transducer for the output of the results of measurement.

7. Device according to any of Claims 1 to 6,
**characterized** in that said display and evaluation circuit terminates the measuring cycle if a rest status is detected again in the blood flow in the skin.

8. Device according to any of Claims 1 to 7,
**characterized** in that said evaluation and output circuit completely or partly switches off the current flowing through said light emitters after the actual measuring phase and following the automatic termination of the measuring cycle.

9. Device according to any of Claims 1 to 8,
**characterized** in that said evaluation and output means stores a plurality of movement programs which it outputs by acoustical means.

10. Device according to any of Claims 1 to 9,
**characterized** in that said evaluation and output means stores a "learning succession of sounds".

11. Device according to any of Claims 1 to 10,
**characterized** in that the optical measuring head is configured as a sensor having optical fibres.

12. Device according to any of Claims 1 to 11,
**characterized** in that said evaluation and output circuit automatically assigns a consecutive and non-resettable number to each measuring cycle.

## Revendications

1. Dispositif à la détection non-invasive d'anomalies périphériques de l'écoulement et du passage du sang dans les extrémités humaines, qui comporte
- au moins un émetteur de lumière et au moins un récepteur de lumière,
- une boucle de régulation (1) à asservir une unité de régulation du niveau du courant qui circule dans ledit ou respectivement lesdits émetteur(s) de lumière,
- ainsi qu'un circuit d'évaluation et de sortie
pour la détection de la variation en fonction du temps du processus de l'expulsion ou respectivement de l'apport du sang aux veines, en mesurant la variation de la réflexion de la lumière,
**caractérisé en ce** que le rapport signal/bruit du signal de sortie dudit ou respectivement desdits émetteur(s) de lumière est appliqué à ladite boucle de régulation comme grandeur de référence pour détecteur la variation en fonction du temps du processus de l'expulsion ou respectivement de l'apport du sang aux veines, et
en ce que ladite unité (3) de régulation du niveau commande ledit ou respectivement lesdits émetteur(s) de lumière de façon que le courant qui circule dans le ou respectivement lesdits émetteur(s) de lumière soit augmenté, avant le début du mesurage au sens propre, jusqu'à ce que le rapport signal/bruit vienne d'atteindre une valeur déterminée juste encore suffisante pour le mesurage, et que cette valeur du courant soit conservée pendant le mesurage.

2. Dispositif selon la revendication 1,
**caractérisé** en ce que ledit circuit d'évaluation et de sortie comprend une boucle de régulation active, commandée par un microprocesseur, à asservir le courant qui circule dans ledit ou respectivement lesdits émetteur(s) de lumière.

3. Dispositif selon la revendication 1 ou 2,
**caractérisé** en ce que ledit circuit d'évaluation et de sortie ne permet le départ d'un cycle de mesurage qu'à la détection d'un état de repos dans l'irrigation sanguine cutanée.

4. Dispositif selon une quelconque des revendications 1 à 2,
**caractérisé** en ce que le courant circulant dans ledit ou lesdits émetteur(s) de lumière est modulé par une fréquence déterminée, et en ce que ledit circuit d'évaluation et de sortie démodule le signal de sorte dudit ou desdits récepteur(s) à la même fréquence.

5. Dispositif selon une quelconque des revendications 1 à 4,
**caractérisé** en ce que ledit circuit d'évaluation et de sortie comprend une unité d'affichage à cristaux liquides.

6. Dispositif selon une quelconque des revendications 1 à 5,
**caractérisé** en ce que ledit circuit d'affichage et de sortie comprend un transducteur électro-acoustique pour la sortie des résultats de mesure.

7. Dispositif selon une quelconque des revendications 1 à 6,
**caractérisé** en ce que ledit circuit d'affichage et de sortie achève le mesurage dès qu'un état de repos dans l'irrigation sanguine cutanée est détecté.

8. Dispositif selon une quelconque des revendications 1 à 7,
**caractérisé** en ce que ledit circuit d'évaluation et de sortie coupe, soit complètement soit en partie, le courant circulant par lesdits émetteurs de lumière après le cycle de mesurage au sens propre et après la coupure automatique du mesurage.

9. Dispositif selon une quelconque des revendications 1 à 8,
**caractérisé** en ce que ladite unité d'évaluation et de sortie met en mémoire une pluralité des programmes de mouvement, qu'elle fait sortir de voie acoustique.

10. Dispositif selon une quelconque des revendications 1 à 9,
**caractérisé** en ce que ladite unité d'évaluation et de sortie met en mémoire une "suite de sons autodidactique".

11. Dispositif selon une quelconque des revendications 1 à 10,
**caractérisé** en ce que la tête de mesure optique est configurée sous forme de détecteur à fibres optiques.

12. Dispositif selon une quelconque des revendications 1 à 11,
**caractérisé** en ce que ledit circuit d'évaluation et de sortie assigne à chaque cycle de mesurage automatiquement un nombre en continu qu'on ne peut pas remettre à l'état initial.
